Europäisches Patentamt

European Patent Office

Office européen des brevets

(11) Publication number: **0 497 399 A1**

# EUROPEAN PATENT APPLICATION

(21) Application number: **92200099.7**

(22) Date of filing: **15.01.92**

(51) Int. Cl.5: **G01N  33/50**

(30) Priority: **28.01.91 US 647379**

(43) Date of publication of application:
**05.08.92 Bulletin  92/32**

(84) Designated Contracting States:
**AT BE CH DE DK ES FR GB GR IT LI LU NL PT SE**

(71) Applicant: **THE PROCTER & GAMBLE COMPANY**
**One Procter & Gamble Plaza**
**Cincinnati Ohio 45202(US)**

(72) Inventor: **Perkins, Mary Ann**
**7230 Fernbank Ave.**
**Cincinnati, OH 45233(US)**
Inventor: **Osborne, Rosemarie (NMN)**
**2575 Indian Creek Road**
**Oxford, OH 45056(US)**

(74) Representative: **Canonici, Jean-Jacques et al**
**Procter & Gamble European Technical**
**Center N.V. Temselaan 100**
**B-1853 Strombeek-Bever(BE)**

(54) **Process for evaluating skin irritancy.**

(57)  Described herein is an in vitro alternative method to replace or augment the Draize rabbit skin irritation test for human skin irritation prediction. This in vitro model consists of a three-dimensional human skin co-culture (HuK/F) of keratinocytes and fibroblasts seeded onto medical grade nylon mesh. A battery of assays predictive of skin irritation using cytotoxicity and pro-inflammatory endpoints are used. These assays are used to evaluate compositions which come into contact with the skin, particularly anionic, cationic and nonionic surfactants, across an array of irritancy potential. For prostaglandin generation and cell viability endpoints, the in vitro co-culture system endpoints correlated well with human skin patch irritation data.

EP 0 497 399 A1

TECHNICAL FIELD

The use of skin cultures for evaluating skin irritancy of ingredients and of commercial products provides a viable alternative method to animal testing. The irritancy is measured in co-cultures of human keratinocytes and fibroblasts using tests of cell viability, cytotoxicity, glucose utilization and release of pro-inflammatory mediator PGE2 (prostaglandin E2).

BACKGROUND OF INVENTION

All commercial products have the potential to produce toxicologic insult. Many diverse products and ingredients come into direct or indirect contact with the skin. An assessment of the potential for these product ingredients and formulations to produce skin irritation is a key element of their safety profile.

Although many approaches to safety evaluation have been developed, most methods use animal models and extrapolate from animal to man. In the case of skin irritation testing, the Draize Rabbit Skin Patch Test has been the historical model of choice to position irritancy potential of materials prior to human exposures. Current socio-political pressures on industry and regulators to ban animal testing mandates that predictive alternative in vitro methods be developed, particularly in the area of skin and eye irritation testing, Thompson, R.C., Reducing the Need for Animal Testing, FDA Consumer, 1988. There is a critical need for in vitro alternative tests to continue to ensure the safety of consumers.

In vitro methods are of interest because they could provide short-term, non-animal alternative methods for evaluating materials for irritancy potential, as well as provide a better understanding of the mechanisms of skin irritation, Rowan, A.N., Perspectives on Alternatives to Current Animal Testing Techniques in Preclinical Toxicology, Ann. Rev. Pharmacol Toxicol., 25:225-47, (1985). Typically, in vitro model development has been an empirical approach, relying on correlations rather than understanding of the structure and function of the organ of interest. However, because of recent advances in methods for culturing of human skin cells, it is now possible to determine the effects of irritants on reconstructed human skin.

A commercial culture system has been developed which is the closest in vitro analog to human skin available. These cultures contain a multi-layered dermis with mitotically and metabolically active dermal fibroblasts, naturally secreted collagen Types I and III, and a basement membrane zone containing laminin and collagen Type IV. A multi-layered epidermis is present in the co-cultures.

The development of a battery of objective quantitative biomarkers of skin irritation utilizing cytotoxicity and irritancy endpoints in human skin cell monolayers and co-culture "skin equivalents" is the focus of the present invention. The predictive endpoints can be used to establish rank orders of agents with known toxicity within a chemical class in order to establish a baseline for assessing and predicting toxicity of related novel agents. Further, the culture system has the potential to predict absolute levels of human skin irritation. The endpoints can also be used to evaluate the utility of commercial sources of human skin cultures. Skin equivalents provide skin toxicity information related to mechanisms of erythema and edema, e.g. PGE2 (prostaglandin E2) release as a proinflammatory mediator. It is likely that irritancy data from these systems will be acceptable to regulatory agencies.

It is an object of this invention to:

1) Provide a battery of assays in in vitro skin culture systems that could be used as a pre-clinical screen for human skin irritation. These assays include measures of cell viability, cytotoxicity, and glucose utilization, as well as release of the pro-inflammatory mediator PGE2.

2) Apply this battery of assays to evaluate human skin culture responses to anionic, cationic and nonionic surfactants and other irritants, in order to rank order irritant potential in vitro and determine correlation with human in vivo skin irritation data.

3) Compare responses of human skin keratinocytes and fibroblasts in monolayer culture to co-cultured human skin fibroblasts/keratinocytes.

These and other objects will become apparent from the description below.

SUMMARY OF THE INVENTION

The present invention includes the evaluation of cell viability by two techniques: 1) incorporation of the vital dye neutral red into lysosomes of viable cells; and 2) reduction of a tetrazolium salt (MTT) to formazan dye by the respiratory electron transport chain of cells with competent mitochondria.

Cytotoxicity endpoints which measure the irritancy potential of surfactants and surfactant-containing products and other materials which come into contact with skin include release of the cytoplasmic enzyme lactate dehydrogenase (LDH) and release of the lysosomal enzyme N-acetylglucosaminidase (NAGS) from damaged cells into culture medium. Prostaglandin E2 (PGE2) can also be measured as a marker of a pro-inflammatory state. Glucose utilization by skin cells is used as an indicator of metabolic activity. Skin sensitivity as well as the effects of ultraviolet light on skin can be measured by the method herein.

## DESCRIPTION OF THE FIGURES

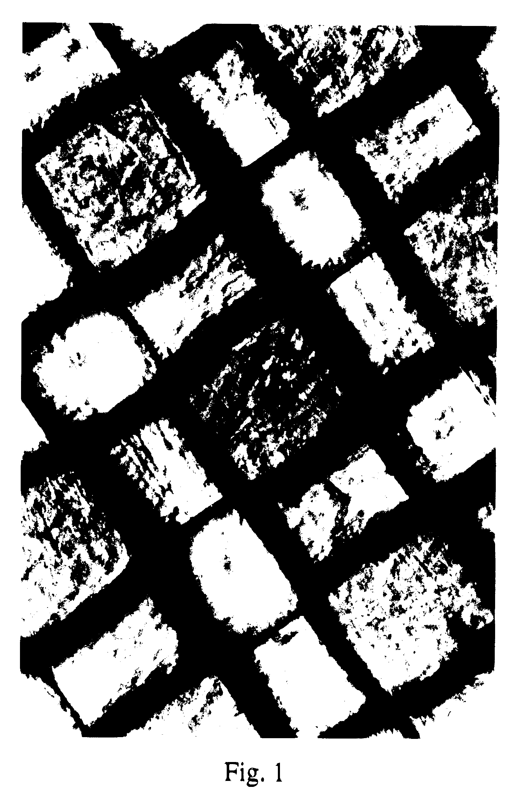

Human skin cell co-cultures consisting of human neonatal foreskin fibroblasts and keratinocytes contain a substratum of fibroblasts grown on a nylon mesh. A view (250x magnification) of the surface of a culture (Fig. 1) indicates the large number of cells that grow in the spaces between the interwoven nylon mesh filaments. Phase contrast microscopy of the mesh surface, as in Fig. 1 was routinely used to confirm the cellularity of the cultures.

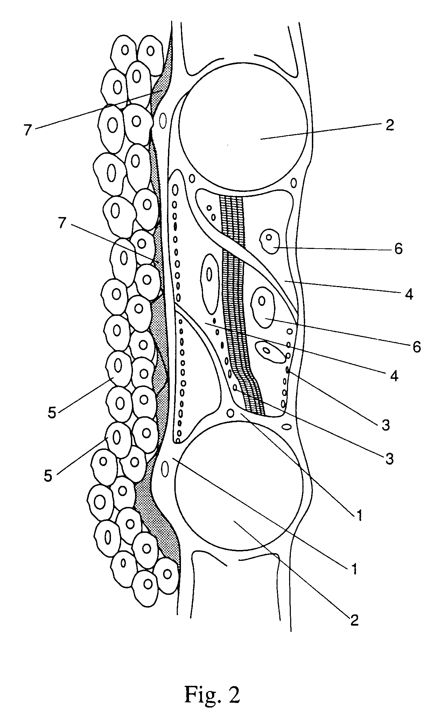

A schematic diagram of a commercial (Marrow-Tech) culture in cross-section (Fig. 2) shows fibroblast (1) attachment to the nylon filaments (2), the presence of collagen (3) and extracellular matrix components (4) secreted from the cells, and the epidermal keratinocytes (5) growing in layers above the fibroblast/nylon substratum. Although adipocytes (6) and melanocytes (7) are included in this diagram, they were not components of the cultures used.

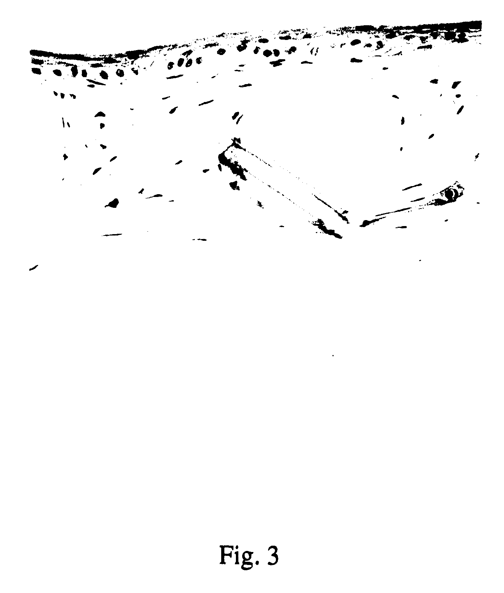

An histological cross-section (400x magnification) of a commercial (Marrow-Tech) culture of the type used in the examples (Fig. 3) shows approximately 5 cell layers of human neonatal foreskin fibroblasts, one layer of basal keratinocytes, and 2-3 layers of flattened stratified keratinocytes on top.

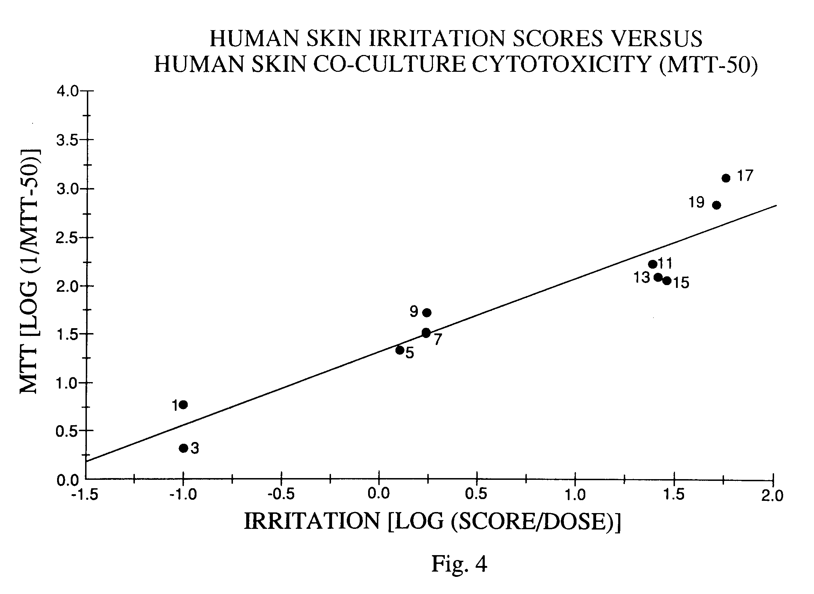

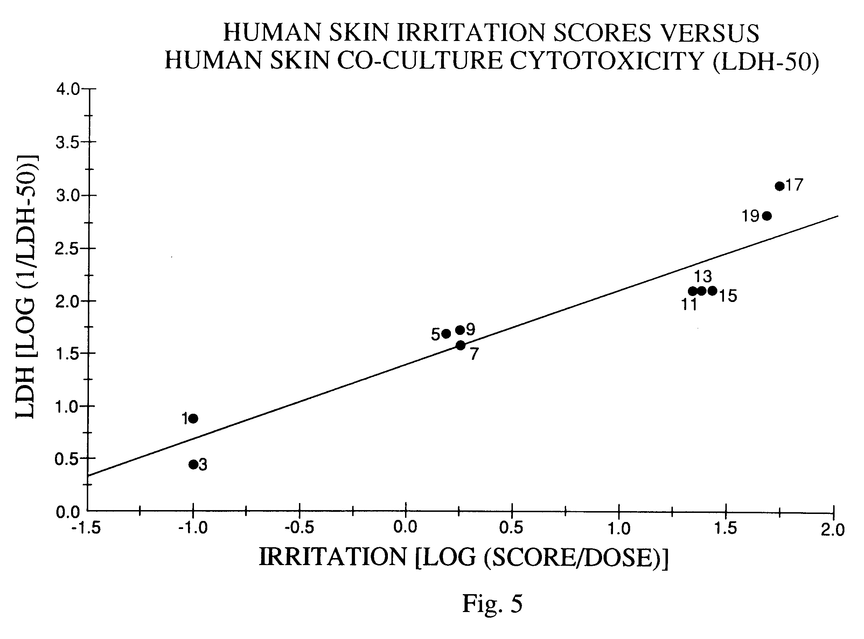

Figure 4 and 5 shows a comparison of the results of human studies with the results of the same materials tested by the method herein. In Figures 4 and 5, the surfactants tested were:

1. Polyoxyethylenesorbitan monolaurate (Tween 20), Sigma Chemical Co., Catalog #P-1379.
3. Polyoxyethylenesorbitan Monooleate (Tween-80), Sigma Chemical Co., Catalog #P-1754.
5. Dodecyl Dimethyl Amine Oxide (C12DDAO), Procter & Gamble, 31.0% active.
7. Ammonium Coconut Alkyl Ethoxylate 6.5 Sulfate (NH4CnAE6.5S), Procter & Gamble, 67.5% active.
9. Ammonium Coconut Alkyl Ethoxylate 12 Sulfate (NH4CnAE12S), Procter & Gamble, 63.91% active.
11. Sodium Dodecyl Sulfate (SDS), Sigma Chemical Co., Catalog #L-4509.
13. C12.3 Alkane Benzene Sulfonate (C12.3 LAS), Procter & Gamble, 96.46% active.
15. C11.8 Alkane Benzene Sulfonate (C11.8 LAS*H), Procter & Gamble, 97.03% active.
17. Benzalkonium Chloride, Sigma Chemical Co., Catalog #B-1383.
19. Benzethonium Chloride, Sigma Chemical Co., Catalog #B-8879.

## DETAILED DESCRIPTION OF THE INVENTION

Cell Cultures: Human skin cell co-cultures consisting of human neonatal foreskin fibroblasts and keratinocytes seeded onto an inert medical grade nylon mesh are used. These are available from Marrow-Tech (10933 North Torrey Pines Road, LaJolla, California 92037). A similar culture grown in a collagen matrix on a filter is available from Organogenesis (83 Rogers Street, Cambridge, Massachusetts 02142). Clonetics (9620 Chesapeake Drive, San Diego, California 92123) has human skin keratinocyte and dermal fibroblast monolayer cell culture models. The cell co-cultures if air-interfaced can contain a stratum corneum layer which resembles skin. This layer has differentiated keratinocyte cells and is a skin equivalent. The skin equivalent cultures are most preferred because they can be used as a model for percutaneous absorption of the challenging surfactant or product containing it.

As used herein, "HuK/F" refers to these human skin neonatal foreskin keratinocyte and fibroblast co-cultures.

As used herein, "HuK" refers to cell cultures of human keratinocytes.

As used herein, "HuF" refers to cull cultures of human fibroblasts.

The human skin co-culture system has advantages over other commercially available skin toxicity testing systems in that it is morphologically similar to human skin. This is particularly true of those which contain a stratum corneum layer. One problem inherent in in vitro models is that cell cultures present physical problems regarding the solubility, stability and biophysical effects of the test compound in the aqueous culture medium in which the cells are grown and treated. Stratum corneum-containing skin cultures allow application of test material to the surface of the cell layer.

Capillary channels containing cultured endothelial cells for feeding the cells and for sampling release of biomarkers could also be incorporated into the culture. Cells that contribute to the inflammatory process such as melanocytes and mast cells could be incorporated into the cultures for better response.

In preliminary experiments the response of HuK/F cultures that contained cells from either neonatal (foreskin) or adult (breast) skin were compared. The neonatal cells gave more consistent responses to sodium dodecyl sulfate than did the adult cells. Mixing experiments suggested that there was donor to donor variability in the ability of the adult dermal fibroblasts to support the growth of keratinocytes. For this reason, neonatal Huk/F is a more consistent model and therefore preferred.

One great advantage in skin irritation toxicology is the availability of human data for comparison with in vitro data. The human skin irritation data is based on a subjective endpoint (i.e., visual skin grading for erythema and edema, 0-4 grades). The data used for our comparisons are from a variety of sources including, Nixon, G.A., Bannon, E.A., Gaynor, T.W., Johnston, D.H. and Griffith, J.F., Evaluation of Modified Methods for Determining Skin Irritation in Animals, Regulatory Toxicol. Pharmacol., 1990, in press, and non-published data from Procter & Gamble. The human scores have been normalized for dose concentration.

The correlation between the cytotoxicity endpoints measured in HuK/F cultures and human skin response shows promise for prediction of human skin responses to the class of materials known as surfactants. See Figures 4 and 5.

The cell cultures can be maintained as follows:

The cultures are placed aseptically into 24 well plates (Corning Catalog #25820) in a medium (2 ml/well) consisting of Dulbecco's Modified Eagle's Medium (Cellgro, Catalog #15-013-LM) to which is added Modified Eagle's Medium with Non-Essential Amino Acids (Gibco Catalog #320-114AG, 1x final concentration), L-Glutamine (Cellgro Catalog #25 -005-L1, 1x final concentration), Antibiotic/Antimycotic (Gibco Catalog #600-5240-AG, 1 x final concentration), and Ultroser-G serum substitute (IBF, Catalog #259501, 1-ten ml bottle/500 ml medium). All cultures are maintained in a humidified atmosphere (Fisher $CO_2$ Incubator Model #610) at $37^0$C and 5% carbon dioxide ($CO_2$) throughout the experiment. Culture medium is aseptically (Baker Hood) changed every two days prior to treatment (2 ml growth medium/well). Usually, the cultures are used for experimentation within one week of arrival from the supplier.

These growth mediums are well known in the art and it is within the skill of the biologists to determine the medium which can be used to maintain cell viability and cell growth. The medium contains essential cell nutrients.

For some tests, co-cultures are not necessary. Human fibroblast cultures or human keratinocyte cultures can be used. Before using these cultures, the cell stocks are treated with trypsin and are subcultured.

Human neonatal fibroblasts are grown in Dulbecco's Modified Eagles Medium (DMEM) supplemented with 10% Fetal Bovine Serum (FBS) (provided by Marrow-Tech). This maintenance medium is the same formulation as described for the above co-cultures with the substitution of 10% FBS for the 2% Ultroser-G medium. Twenty-four hours prior to treatment fibroblasts are trypsinized (Gibco, Trypsin EDTA, Catalog #610-5200AG) by placing 2 ml of 0.25% trypsin over PBS washed cells. After about 1 minute the trypsin is aspirated and cells observed microscopically for rounding up and release from the culture dish. Released cells are washed into a 50 ml conical tube with 10% FBS/DMEM to neutralize the trypsin, and centrifuged at 200 g for 10 minutes. The medium is aspirated and the cell pellet resuspended for counting on the Coulter Multisizer. Cells are diluted further with 10% FBS-DMEM and plated in a 24 well Corning culture plate (about 33,000 cells/test well).

Human neonatal foreskin keratinocytes are grown in keratinocyte growth medium-KGM complete with epidermal growth factor, insulin, hydrocortisone, anti-microbial agents and supplemented with bovine pituitary extract. (This medium is available from Clonetics). Cultures are maintained in a humidified atmosphere at $37^0$C and 5% carbon dioxide throughout the experiment. Four days prior to treatments keratinocytes are trypsinized and subcultured. KGM medium is used and Clonetics reagents and methods used as per their standard protocol. Cells are plated at a density of about 33,000/ well in a 24 well Corning culture plate.

Some of the endpoints selected for evaluation in the skin model have been shown to be relevant by other investigators in animal and human skin and skin cultures, Gibson, W.T. and Teal, M.R., Interactions of C12 Surfactants with the Skin: Changes in Enzymes and Visible Histological Features of Rat Skin Treated with Sodium Lauryl Sulfate, Fd Chem Toxic 21:587-594, 1983 and Csato', M., Rosenbach, T, Grabbe, J. and Czarnetski, B.M., Epidermal Scales; Are They a Wastebasket of Inflammatory Mediators or Active Participants in Epidermal Inflammation?, Int J of Derm 28:86-89, 1989.

As used herein, "PGE2" means prostaglandin E2.

In the case of PGE2, our studies have shown that in a mouse skin model this eicosenoid could be detected in intact mouse skin treated with sodium dodecyl sulfate using a unique non-invasive sampling technique. Human skin clinical tests using the same sampling technique showed that PGE2 levels in human skin increased with surfactant exposure. This data indicates that PGE2 may be an excellent in vitro biomarker that has direct relevance to human clinical results. The stimulation of PGE2 production in response to the surfactants shows that the cultures are useful for studying molecular and cellular pathways of irritant-induced skin inflammation and therefore provide a mechanistic approach to assessing skin irritation.

The development of a battery of objective quantitative biomarkers of skin irritation utilizing cytotoxicity and irritancy endpoints in human epidermal keratinocytes (HuK), dermal fibroblasts (HuF), and HuK/F co-cultures or "skin equivalents" is based on the evaluation of cells and culture medium from skin cultures treated with surfactants, important ingredients in many cleaning and skin care products.

MTT assay for cell viability (based on the reduction of a tetrazolium dye by functional mitochondria) is preferred to the neutral red assay (hereinafter referred to as, "NR") which is based on incorporation of NR dye into the lysosomes of viable cells. The preference is due to greater maximal incorporation of MTT, and a lower nonspecific binding of dye to nylon meshes for MTT versus NR.

Responses to a prototype surfactant, sodium dodecyl sulfate, are seen as early as 4 hours after treatment, are maximal at 24 to 48 hours, and are maintained for up to 72 hours. The "effective time of treatment" is the amount of time needed to see a result. This will usually be from 4 hours to 72 hours. The maximum results are usually evident at 24 to 48 hours.

In HuK/F cultures treated for 48 hours, there are dose dependent changes in cell viability (MTT incorporation), cytotoxicity (lactate dehydrogenase-LDH and N-acetylglucosaminidase-NAGS release), glucose utilization and prostaglandin E2 (PGE2) generation in response to the materials tested. There is a close correlation among the endpoints for responses to the surfactants, and between the in vitro responses and human skin irritation responses to the surfactants (Figures 4 and 5). HuK and HuF cells responded to sodium dodecyl sulfate with dose-dependent decreases in cell viability although these cells were approximately 10-fold more sensitive than the HuK/F cultures. These data indicate that keratinocytes and fibroblasts are each responsible in part for the responses observed in the HuK/F co-cultures.

The method described herein can be used to test a variety of materials which come into contact with the skin. These include surfactants (anionic, cationic, or nonionic), and products containing these surfactants, e.g. shampoos, detergents, fabric softeners, conditioners, dishwashing liquids, skin cleansers, cleaning agents and skin care items. Other materials and products that come into contact with the skin can also be tested. These include permanent waving solutions, hair straighteners, hair dyes, cosmetics, moisturizers, colors and dyes used in cosmetics, cleaning agents, sunscreens and tanning agents. These materials all have a potential to cause skin irritation.

The following description of the assays and testing method is illustrated with surfactants. However, as is obvious from the methodology description, it can be applied to the other ingredients or products which contact the skin.

Test Material Preparation

A positive control of 0.01% sodium dodecyl sulfate salt (sodium dodecyl sulfate, Sigma #L-4509) is included as a positive internal control for each test plate. One hundred milligrams of sodium dodecyl sulfate is dissolved, with vortexing, in 10 ml of sterile deionized distilled water (Lab-5-Technic) and sterile filtered using a 0.2 micron filter (Nalgene, 150 ml). The 1% stock is diluted (1:100) with the appropriate amount of medium to prepare a 0.01% sodium dodecyl sulfate treatment solution. Sterile stock solutions of each test material are prepared in culture medium. Samples are vortexed at high speed or sonicated, as required, to dissolve the test materials, and then pre-warmed to $37^0$C before cell treatment.

Test solution dilutions are prepared in Marrow-Tech or similar cell medium from a sterile stock solution at the appropriate dilutions. The pH (Orion Model EA920 pH electrode) and osmolarity (measured with Wescor 5500 vapor pressure osmometer) of the highest concentration of test solution is recorded.

Treatments

For each HuK/F experiment, on a 24 well plate, 1 test material is evaluated at 4 concentrations (in quadruplicate wells per each concentration). In addition, 4 wells serve as medium control, and 4 wells as sodium dodecyl sulfate positive control. For each material evaluated, a dose-range finding study is performed at 0.001, 0.01, 0.1 and 1.0% (w/v) concentrations to determine the concentration eliciting minimum and maximum cytotoxicity in the MTT assay of cell viability. Based on the dose-range finding study, a range of test material concentrations is selected to determine the concentration eliciting minimum and maximum cytotoxicity in the MTT assay of cell viability. Based on the dose-range finding study, concentrations are selected at the maximal cytoxicity level and minimum level and at two 1/3 log intervals between these concentrations (e.g., if the maximum cytoxicity was at 1% with no cytotoxicity or cell death at 0.1% the concentrations evaluated subsequently would be 0.1, 0.3, 0.6 and 1%).

On treatment day, growth medium is aseptically aspirated, and the cell meshes are treated with two ml

EP 0 497 399 A1

of test medium per well. Medium alone served as a no treatment control with 0.01% sodium dodecyl sulfate acting as a positive control (n = 4 wells/group). All test plates are incubated at $37^0$C and 5% carbon dioxide for 4 to 72 hours. Observations are made (e.g. changes in medium color) during the treatment period.

Sample Collection and Analysis

After the treatment period (generally 48 hours) the medium samples are collected (2/ml per well) and split into two one ml aliquots in individually labeled cryotubes (available from Corning Catalog #25702). A one ml sample is analyzed for lactate dehydrogenase, N-acetylglucosaminidase, and glucose using Boehringer-Mannheim biochemicals automated on an Hitachi 705 autoanalyzer. A second one ml sample is purged with nitrogen, immediately placed on dry ice and stored frozen at $-70^0$C. These samples are subsequently analyzed for Prostaglandin E2 by radioimmunoassay (Advanced Magnetics kit #6001).

Cell Viability Assays

In preliminary experiments with HuK/F co-cultures, two vital dye assays measuring cell viability can be used. The Neutral Red Assay measures the uptake of neutral red into lysosomes of viable cells. The MTT assay measures the reduction of a tetrazolium dye by electron transport in mitochondria of viable cells, and subsequent intracellular trapping of the formazan product.

1. The MTT Assay was adapted from a method described in Mossmann, T., Rapid Colorimetric Assay for Cellular Growth and Survival: Application to proliferation and cytotoxicity assays, Journal of Immunological Methods: 65:55-63, (1983). It is a measure of cell viability, and is performed on all treatment meshes immediately following medium collection. The MTT assay quantitates the reduction and subsequent trapping of a yellow tetrazolium dye which is reduced by the electron transport chain of functional mitochondria to a purple formazan dye. Briefly, MTT (3-[4,5-Dimethylthiazol-2yl]-2,5-diphenyltetrazolium bromide; Sigma Catalog #M-2128) powder is diluted in cell culture medium (0.5 mg./ml.) and pre-warmed to $37^0$C before use. One ml of this dye solution is aliquoted onto each mesh, and the meshes are then incubated 3 hours at $37^0$C and 5% $CO_2$. After incubation, the MTT is aspirated, and 2 ml. of pure isopropanol is used to extract the reduced dye for two hours at room temperature. The meshes are removed from the culture well and extraction solvent mixed by stirring before aliquoting for absorbance readings. The absorbance of each extract is read at 540 nm on a Biotek EL-312 (96 well) spectrophotometer.

2. The Neutral Red Bioassay is described in Borenfreund, E., and Puerner, J., A Simple Quantitative Procedure Using Monolayer Cultures for Cytotoxicity Assays (HTD/NR-90), J. Tissue Cult. Meth. 9(1):7-9, (1984). It is designed to measure quantitatively the toxicity of test agents to Normal Human Epidermal Keratinocytes (NHEK or HuK) grown under serum free conditions. Neutral Red is a water soluble dye that passes through the plasma membrane of the cell and becomes incorporated into the lysosomes of living cells. Neutral Red (3-amino-7-dimethylamino-2-methylphenazine hydrochloride, Sigma Catalog #N2889), is prepared in cell culture medium for co-cultures, or in the case of keratinocyte monolayer cells in the KGM medium (50 $\mu$g./ml). The cells are evaluated using the standard neutral red assay methodology. For co-cultures this assay is performed as described by Dr. Borenfreund but with modifications as described above for the MTT assay, e.g. removal of meshes.

Neutral Red incorporation (NR) was compared to MTT in control cultures and cultures treated with 0.01% sodium dodecyl sulfate or 1% concentrations of benzalkonium chloride, benzethonium chloride, and Tween 20. These concentrations were selected as maximally cytotoxic. Under the conditions used (linear for both assays) comparisons of dye incorporation based on absorbance readings indicated MTT incorporation was 5-6 fold greater than NR in control cultures. In surfactant-treated cultures, NR incorporation was 2.2 fold greater than MTT; this level of NR or MTT incorporation was equivalent to background binding of dye to mesh alone (without cells). The MTT assay is preferred to the NR assay because of the greater dynamic range of the assay (due to higher maximal incorporation of dye and lower non-specific binding to the nylon mesh substrate).

In order to determine the optimal time for treatment of the cultures, time-course experiments examining the responses of HuK/F cultures to sodium dodecyl sulfate were conducted. Significant responses were seen at 4 hours after treatment. HuK/F responses to sodium dodecyl sulfate for MTT uptake, glucose utilization, and PGE2 synthesis were near maximal at 24 hours, and were maintained or greater at 48 hours. Forty-eight hours is a preferred treatment period because, in general, maximal responses were produced at this time.

In HuK/F cultures, there were dose dependent changes in cell viability (MTT incorporation), cytotoxicity,

6

glucose utilization and PGE2 generation in response to the surfactants tested. In the case of PGE2 it was determined that higher concentrations of some surfactants can interfere with this analysis. Other biochemical endpoints evaluated in medium of treated cells included measuring total protein, alkaline phosphatase, acid phosphatase, calcium, potassium and sodium concentrations, but these endpoints did not change in response to chemical exposure.

For MTT and LDH endpoints there was good agreement with human skin irritation responses to the surfactants (Figures 4 and 5).

The human keratinocyte monolayer cultures (available from Clonetics) were characterized using a fluorescein-labeled anti-cytokeratin antibody that stained the cytoskeleton of keratinocytes preferentially. This stain confirms that the cells are epithelial in nature. HuK and HuF cells responded to sodium dodecyl sulfate with a dose-dependent decrease in cell viability as demonstrated by the NR assay, and are about 10-fold more sensitive than HuK/F cultures. PGE2 release in HuK cells was significantly increased about 2.5 fold over control. These cells were approximately 10-fold more sensitive than the HuK/F cultures for PGE2 release.

Tables 1 through Table 3 demonstrate the use of these methods on various surfactants.

TABLE 1

| MTT-50 Calculations for Test Materials | |
|---|---|
| Surfactant | MTT-50 Endpoint (Concentration w/v) |
| Benzalkonium Chloride | 0.0007% |
| Benzethonium Chloride | 0.0014% |
| Sodium Dodecyl Sulfate | 0.0057% |
| C12.3 LAS*H | 0.0070% |
| C11.8 LAS | 0.0073% |
| C12 DDAO | 0.018% |
| NH4CNAE6.5S | 0.019% |
| NH4CNAE12S | 0.025% |
| Tween 20 | 0.18% |
| Tween 80 | 0.43% |

## TABLE 2
### Lactate Dehydrogenase Release (U/L)

| Surfactant | Control | 0.01 | 0.03 | 0.06 | 0.1 |
|---|---|---|---|---|---|
| NH4CNAE6.5S | | | | | |
| Avg | 35.5 | 43.5 | 1637.0+ | 1586.0+ | 1644.0+ |
| NH4CNAAE12S | | | | | |
| Avg | 43.3 | 41.8 | 1005.5+ | 1414.0+ | 1487.5+ |
| C12 DDAO | | | | | |
| Avg | 50.3 | 116.0+ | 1270.0+ | 1261.5+ | 1196.0+ |

| | Control | 0.1 | 0.3 | 0.6 | 1.0 |
|---|---|---|---|---|---|
| Tween 20 | | | | | |
| Avg | 13.8 | 264.3+ | 1404.3+ | 1503.5+ | 1495.5+* |
| Tween 80 | | | | | |
| Avg | 46.3 | 130.0 | 335.0+ | 1022.5+* | 1105.8+ |

| | Control | 0.001 | 0.003 | 0.006 | 0.01 |
|---|---|---|---|---|---|
| SDS | | | | | |
| Avg | 92.5 | 47.5+ | 62.8 | 796.0+ | 1496.0+ |
| Benzethonium Chloride | | | | | |
| Avg | 22.0 | 433.5+ | 1408.0+* | 1344.0+* | 1471.5+ |

| | Control | 0.0005 | 0.001 | 0.005 | 0.01 |
|---|---|---|---|---|---|
| Benzalkonium Chloride | | | | | |
| Avg | 34.0 | 138.5+ | 1032.0+* | 1334.5+* | 1236.5+* |

| | Control | 0.001 | 0.003 | 0.006 | 0.01 |
|---|---|---|---|---|---|
| C11.8 LAS | | | | | |
| Avg | 48.5 | 54.5 | 62.0 | 371.3+ | 1459.5+ |
| C12.3 LAS*H | | | | | |
| Avg | 32.3 | 29.3 | 38.0 | 389.3+ | 1584.0+ |

*3 Samples were used, not 4.

+Indicates a statistically significant difference from control ($p < 0.05$).

## TABLE 3

### N-Acetylglucosaminidase Release (U/L)

#### Treatment (% Concentration W/V)

| Surfactant | Control | 0.01 | 0.03 | 0.06 | 0.1 |
|---|---|---|---|---|---|
| NH4CNAE6.5S | | | | | |
| Avg | 14.38 | 14.28 | 28.30+ | 31.50+ | 24.73+ |
| NH4CNAE12S | | | | | |
| Avg | 14.40 | 14.13 | 29.15+ | 31.15+ | 29.8+ |
| C12 DDAO | | | | | |
| Avg | 17.03 | 21.15 | 23.53+ | 23.33+ | 26.90+ |

| | Control | 0.1 | 0.3 | 0.6 | 1.0 |
|---|---|---|---|---|---|
| Tween 20 | | | | | |
| Avg | 15.45 | 20.45+ | 41.88+ | 42.85+ | 40.85+* |
| Tween 80 | | | | | |
| Avg | 14.03 | 22.08+ | 34.88+ | 43.18+* | 43.33+ |

| | Control | 0.001 | 0.003 | 0.006 | 0.01 |
|---|---|---|---|---|---|
| SDS | | | | | |
| Avg | 13.93 | 14.05 | 14.00 | 17.28+ | 16.58+ |
| Benzethonium Chloride | | | | | |
| Avg | 14.65 | 21.20+ | 37.63+* | 37.10+* | 21.73+* |

| | Control | 0.0005 | 0.001 | 0.005 | 0.01 |
|---|---|---|---|---|---|
| Benzalkonium Chloride | | | | | |
| Avg | 13.98 | 14.95+ | 18.08+* | 19.93+* | 11.98+* |

| | Control | 0.001 | 0.003 | 0.006 | 0.01 |
|---|---|---|---|---|---|
| C11.8 LAS | | | | | |
| Avg | 13.03 | 11.8 | 12.58 | 13.75 | 17.18+ |
| C12.3 LAS*H | | | | | |
| Avg | 14.65 | 13.95 | 14.80 | 16.85 | 21.43+ |

Tables 4 through Table 7 demonstrate the use of these measurements on various commercial surfactant containing compositions. All analyses were done on 4 samples, the values represent averages of the 4 samples. Sodium dodecyl sulfate was included as a positive control.

## TABLE 4

### MTT Viability Assay Absorbance (540nm)

#### Treatment (% Concentration W/V)

| Formulation | Control | 0.001 | 0.01 | 0.1 | 1.0 |
|---|---|---|---|---|---|
| Liquid Detergent A | 1.3498 | 1.2350 | 1.2535 | 0.0683+ | 0.0615+ |

| | Control | 0.01 | 0.03 | 0.06 | 0.1 |
|---|---|---|---|---|---|
| Liquid Detergent A | 1.4605 | 1.3925 | 1.1613 | 0.0615+ | 0.0613+ |

| | Control | 0.001 | 0.003 | 0.006 | 0.01 | 0.03 |
|---|---|---|---|---|---|---|
| Sodium Dodecyl Sulfate | 1.2450 | 1.4313 | 1.5723+ | 1.5820+ | 0.0768+ | 0.0653+ |

| | Control | 0.001 | 0.01 | 0.1 | 1.0 |
|---|---|---|---|---|---|
| Liquid Detergent B | 0.9997 | 1.1538 | 1.3233 | 1.0875 | 0.0650+ |
| Bar Soap A | 1.6960 | 1.6700 | 1.3368 | 0.8510+ | 0.1005+ |
| Bar Soap B | 1.5055 | 1.1750 | 1.4715 | 0.0920+ | 0.0698+ |
| Shampoo A | 1.1190 | 0.8563 | 1.0930 | 0.0778+ | 0.0773+ |
| Shampoo B | 0.9843 | 0.7638 | 0.6215 | 0.0593+ | 0.0638+ |
| Liquid Detergent C | 1.0603 | 1.0865 | 1.2018 | 0.0787+ | 0.0725+ |

| | Control | 0.01 | 0.03 | 0.06 | 0.1 |
|---|---|---|---|---|---|
| Liquid Detergent C | 1.1720 | 1.1795 | 0.8773 | 0.0760+ | 0.0728+ |

| | Control | 0.001 | 0.01 | 0.1 | 1.0 |
|---|---|---|---|---|---|
| Shampoo A | 1.0158 | 1.3185 | 1.5805 | 0.0658+ | 0.0658+ |

| | Control | 0.03 | 0.06 | 0.1 | 0.6 | 1.0 |
|---|---|---|---|---|---|---|
| Shampoo A | 1.4770 | 1.6673 | 0.0945+ | 0.0608+ | 0.0600+ | 0.0585+ |

| | Control | 0.001 | 0.01 | 0.1 | 1.0 |
|---|---|---|---|---|---|
| Granular Detergent A | 1.1160 | 1.1102 | 1.0543 | 0.2430+ | 0.0658+ |

| | Control | 0.01 | 0.03 | 0.06 | 0.1 |
|---|---|---|---|---|---|
| Granular Detergent A | 1.1633 | 0.9563 | 1.1100 | 0.0758+ | 0.0740+ |

## TABLE 5

### Lactate Dehydrogenase Release (U/L)

#### Treatment (% Concentration W/V)

| Formulation | Control | 0.001 | 0.01 | 0.1 | 1.0 |
|---|---|---|---|---|---|
| Liquid Deter- gent A | 18.3 | 20.5 | 15.3 | 513.8+ | 0.00+ |

| Formulation | Control | 0.01 | 0.03 | 0.06 | 0.1 |
|---|---|---|---|---|---|
| Liquid Deter- gent A | 34.8 | 51.0 | 77.0 | 934.5+ | 962.3+ |

| Formulation | Control | 0.001 | 0.003 | 0.006 | 0.01 | 0.03 |
|---|---|---|---|---|---|---|
| Sodium Dodecyl Sulfate | 24.5 | 24.0 | 29.8 | 101.0+ | 754.0+ | 1.3+ |

| Formulation | Control | 0.001 | 0.01 | 0.1 | 1.0 |
|---|---|---|---|---|---|
| Liquid Deter- gent B | 18.0 | 17.0 | 16.0 | 17.3 | 32.3 |
| Bar Soap A | 26.0 | 22.8 | 25.0 | 261.3+ | 28.5 |
| Bar Soap B | 20.3 | 19.3 | 21.5 | 405.0+ | 5.8+ |
| Shampoo A | 23.0 | 22.0 | 23.5 | 358.8+ | 0.0+ |
| Shampoo B | 19.3 | 23.0 | 162.0+ | 277.8+ | 0.0+ |
| Liquid Deter- gent C | 61.5 | 54.5 | 58.3 | 522.0+ | 0.0+ |

| Formulation | Control | 0.01 | 0.03 | 0.06 | 0.1 |
|---|---|---|---|---|---|
| Liquid Deter- gent C | 41.3 | 41.8 | 400.0+ | 816.8+ | 200.3+ |

| Formulation | Control | 0.001 | 0.01 | 0.1 | 1.0 |
|---|---|---|---|---|---|
| Shampoo C | 17.3 | 14.3 | 13.5 | 296.8+ | 0.0+ |

| Formulation | Control | 0.03 | 0.06 | 0.1 | 0.6 | 1.0 |
|---|---|---|---|---|---|---|
| Shampoo C | 27.5 | 64.3+ | 732.3+ | 483.5+ | 0.3+ | 0.0+ |

| Formulation | Control | 0.001 | 0.01 | 0.1 | 1.0 |
|---|---|---|---|---|---|
| Granular Detergent | 12.0 | 13.0 | 15.8 | 503.0+ | 0.0+ |

| Formulation | Control | 0.01 | 0.03 | 0.06 | 0.1 |
|---|---|---|---|---|---|
| Granular Detergent | 36.5 | 36.3 | 225.3+ | 895.3+ | 586.0+ |

## TABLE 6

### N-Acetyl Glucosaminidase Release (U/L)

#### Treatment (% Concentration W/V)

| Formulation | Control | 0.001 | 0.01 | 0.1 | 1.0 |
|---|---|---|---|---|---|
| Liquid Deter-gent A | 11.35 | 11.30 | 11.45 | 14.13+ | 8.70+ |

| | Control | 0.01 | 0.03 | 0.06 | 0.1 |
|---|---|---|---|---|---|
| Liquid Deter-gent A | 11.80 | 11.38 | 11.33 | 16.15+ | 15.30+ |

| | Control | 0.001 | 0.003 | 0.006 | 0.01 | 0.03 |
|---|---|---|---|---|---|---|
| Sodium Dodecyl Sulfate | 11.25 | 10.88 | 11.45 | 12.95+ | 16.65+ | 9.35+ |

| | Control | 0.001 | 0.01 | 0.1 | 1.0 |
|---|---|---|---|---|---|
| Liquid Deter-gent B | 11.98 | 12.13 | 11.95 | 11.98 | 10.25+ |
| Bar Soap B | 11.18 | 12.18 | 11.93 | 13.65+ | 4.18+ |
| Bar Soap B | 11.40 | 11.40 | 11.53 | 11.25 | 3.17+ |
| Shampoo A | 11.13 | 11.07 | 10.95 | 11.15 | 8.50+ |
| Shampoo B | 11.08 | 11.23 | 11.93+ | 11.23 | 8.57+ |
| Liquid Deter-gent C | 11.75 | 12.10 | 11.68 | 12.40 | 8.40+ |

| | Control | 0.01 | 0.03 | 0.06 | 0.1 |
|---|---|---|---|---|---|
| Liquid Deter-gent C | 11.53 | 11.45 | 14.00+ | 15.43+ | 10.83+ |

| | Control | 0.001 | 0.01 | 0.1 | 1.0 |
|---|---|---|---|---|---|
| Shampoo A | 11.80 | 11.80 | 11.48 | 13.33+ | 8.80+ |

| | Control | 0.03 | 0.06 | 0.1 | 0.6 | 1.0 |
|---|---|---|---|---|---|---|
| Shampoo A | 11.93 | 13.10+ | 16.03+ | 13.32+ | 8.85+ | 8.43+ |

| | Control | 0.001 | 0.01 | 0.1 | 1.0 |
|---|---|---|---|---|---|
| Granular Detergent A | 11.55 | 11.53 | 11.35 | 16.80+ | 8.95+ |

| | Control | 0.01 | 0.03 | 0.06 | 0.1 |
|---|---|---|---|---|---|
| Granular Detergent A | 11.90 | 11.13 | 12.18 | 14.68+ | 13.38+ |

## TABLE 7
### Evaluation of Detergent Formulations in the Marrow-Tech
### Full Thickness Skin Cultures
### Glucose Utilization (MG/DL)
### Treatment (% Concentration W/V)

| Formulation | Control | 0.001 | 0.01 | 0.1 | 1.0 |
|---|---|---|---|---|---|
| Liquid Detergent A | 270.8 | 268.0 | 239.8 | 450.8+ | 457.3+ |

| | Control | 0.01 | 0.03 | 0.06 | 0.1 |
|---|---|---|---|---|---|
| Liquid Detergent A | 211.5 | 175.5+ | 217.8 | 389.5+ | 405.0+ |

| | Control | 0.001 | 0.003 | 0.006 | 0.01 | .03 |
|---|---|---|---|---|---|---|
| Sodium Dodecyl Sulfate | 242.8 | 241.5 | 226.8 | 259.3 | 397.3+ | 415.5+ |

| | Control | 0.001 | 0.01 | 0.1 | 1.0 |
|---|---|---|---|---|---|
| Liquid Detergent B | 268.5 | 259.5 | 227.0 | 258.5 | 417.3+ |
| Bar Soap A | 270.8 | 263.5 | 266.3 | 298.0 | 391.5+ |
| Bar Soap B | 237.8 | 264.3+ | 246.3 | 397.0+ | 339.8+ |
| Shampoo A | 253.5 | 279.0 | 236.5 | 416.3+ | 422.5+ |
| Shampoo B | 280.0 | 293.5 | 325.0+ | 425.8+ | 427.3+ |
| Liquid Detergent C | 248.8 | 248.8 | 247.3 | 409.8+ | 411.8+ |

| | Control | 0.01 | 0.03 | 0.06 | 0.1 |
|---|---|---|---|---|---|
| Liquid Detergent C | 243.5 | 236.3+ | 279.0 | 402.3+ | 418.8+ |

| | Control | 0.001 | 0.01 | 0.1 | 1.0 |
|---|---|---|---|---|---|
| Shampoo C | 260.8 | 257.5 | 248.3 | 423.0+ | 430.3+ |

| | Control | 0.03 | 0.06 | 0.1 | 0.6 | 1.0 |
|---|---|---|---|---|---|---|
| Shampoo C | 237.0 | 258.8 | 374.3+ | 411.8+ | 425.5+ | 425.3+ |

| | Control | 0.001 | 0.01 | 0.1 | 1.0 |
|---|---|---|---|---|---|
| Granular Detergent A | 262.8 | 263.3 | 273.8 | 341.0+ | 436.5+ |

ANALYTICAL METHODS

MTT Assay Protocol For 24 Well Full Thickness Skin Cultures on Mesh

1. Place one untreated, precut mesh (no cells) into one or more wells to determine the background non-specific binding (NSB) of MTT to mesh and to act as absolute blank. In remaining wells place 1 mesh per well of the fibroblast/keratinocyte co-cultures. Add 2 ml of the assay medium which is supplied with the test kit. (Marrow-Tech supplies a medium).

2. Remove medium from each well and add 2 ml/well of test agent diluted in assay medium. Refeed untreated control mesh and mesh without cells with assay medium.

3. Incubate 48 hours at 37⁰C in 5%. carbon dioxide atmosphere.

4. Prepare a solution of 0.5 mg/ml MTT (Sigma Chemical Co. Catalog #M-2128) in assay medium. Centrifuge MTT/medium for 5 minutes at 3000 RPM to pellet undissolved crystals, then prewarm MTT in a 37⁰C waterbath.

5. Remove spent medium. Use 1 ml aliquot to analyze for lactate dehydrogenase and N-acetylglucosaminidase release and glucose; 1 ml can be saved under nitrogen at -70⁰C for PGE2 analysis at a later time.

6. Add 1 ml/well MTT/medium solution. Add medium without MTT to absolute blank well. Incubate cultures at 37⁰C, 5% carbon dioxide for 3 hours.

7. Remove MTT/medium and wash each mesh 2 times with 1 ml Dulbecco's phosphate buffered saline with calcium and magnesium (PBS). About 2-5 minutes each wash.

8. Remove second PBS wash and add 2 ml isopropanol/well at room temperature 2 hours. This will extract the formazan from mitochondria of cells into the supernatant. (Note: the amount of MTT taken up by a cell culture and subsequently released by solvent extraction is proportional to the number of viable cells within the culture). Remove meshes from test well and stir on shaking vortex mixer.

9. Transfer 200 microliter aliquots of the blue spent solvent from each well to a 96 well microtiter plate and read absorbance at 540 nm blanking to absolute blank (well not exposed to the MTT). The wells containing the NSB meshes should be subtracted from the test well absorbances before performing calculations below.

MTT-50 and NR-50 Calculations. The endpoint of the MTT and NR cytotoxicity assays used for reporting the toxicity of test agents is the concentration of test agent which results in a 50% decrease in either MTT or NR dye uptake (i.e., MTT-50 or NR-50) when compared to untreated control values. Calculations were performed as follows:

1) Calculate the mean OD 540 (optical density at 540 nm) of the untreated control wells. Note: When assays were performed on culture mesh, a non-specific binding (NSB) control was evaluated and this value subtracted from each control and treatment absorbance.

2) Calculate the mean 0D 540 of the four wells for each concentration of test agent.

3) Calculate the % of UNTREATED CONTROL for each mean OD 540:

$$\underline{\text{Mean OD 540 of test agent dilution}} \quad \text{x 100} = \text{\% of un-}$$
$$\text{Mean OD 540 of untreated control} \qquad \text{treated control}$$

On a semi-log graph, the % of Untreated Control was plotted on the y axis and the concentration of test agent on the x axis to establish a dose response curve for each test agent. A calculation using a similar triangle method incorporated into a statistical program was performed far each test agent dose response curve to determine the NR-50 or MTT-50 treatment concentration. The NR-50 or MTT-50 represents the concentration of test material (X-axis) where the cell viability was decreased by 50% of the untreated control value.

The MTT and NR assays measure the number of cells which are viable. The cells are treated with a number of doses of challenging agent, i.e. a surfactant, to get a range of responses, from little or no effect to killing of all the cells. The MTT-50s for test agents within a chemical class may be used to rank order their relative toxicities.

Statistics

Statistics were performed using analysis of variance techniques. Provided that Bartlett's test of homogeneity of variance was not significant, treated groups were compared to control group or to one another using the least significant difference (LSD) criterion. When Bartlett's test was significant, comparisons were made using Wilcoxon's rank sum test and a t-test technique which makes allowance for unequal variances. All statistical tests were conducted at a 5%, two sided risk level.

Other Uses

The use of the cell viability and the release of lactate dehydrogenase, N-acytylglucoseaminidase, and glucose from the cell co-cultures can also be used to measure effects of other irritants or materials to the skin. For example, this system can be used for measuring the effect of UV (ultraviolet) radiation, the effect of UV light as a non-chemical irritant, or for phototoxicity screening. The system can also be used to study skin sensitization, age sensitivity, or body site differences in responses of human skin to irritants.

Percutaneous absorption studies and cell proliferation studies, can also be studied using this method. The cell proliferation studies would be a short term assay predictive of skin carcinogenicity.

**Claims**

1. A method for measuring the irritation potential of a composition comprising:
   (1) challenging a human skin keratinocyte and fibroblast cell co-culture with a test material, preferably wherein the test material is a surfactant selected from the group of anionic. nonionic and cationic surfactants, for from 4 to 24 hours; and
   (2) measuring cell viability by reduction of tetrazolium salt to formazan dye (MTT).

2. A method for measuring the irritation potential of a composition comprising:
   (1) challenging a human skin keratinocyte and fibroblast cell co-culture with a test material, preferably, wherein the test material is a surfactant selected from the group of anionic, nonionic and cationic surfactants, for from 4 to 24 hours; and
   (2) measuring cell viability by incorporating neutral red dye into the cells.

3. A method according to claim 1 or 2 wherein the release of lactate dehydrogenase into the cell medium is also measured.

4. A method according to claim 1, 2 or 3 wherein the release of N-acetylglucoseaminidase into the cell medium is also measured.

5. A method according to claim 1, 2, 3, or 4 wherein glucose utilization is measured.

6. A method according to claim 1, 2, 3, 4, or 5 wherein the cell co-culture also has a stratum corneum made of differentiated keratinocyte cells.

7. A method for measuring the irritation potential of a composition comprising:
   (1) challenging a human skin keratinocyte or fibroblast cell culture with a test material for an effective amount of time; and
   (2) measuring cell viability by incorporating neutral red dye into the cells.

8. A method for measuring the irritation potential of a composition comprising:
   (1) challenging a human skin keratinocyte or fibroblast cell culture with a test material for an effective amount of time; and
   (2) measuring cell viability by reduction of tetrazolium salt to formazan dye (MTT).

9. A method according to claim 1,2,3,4,5,6,7, or 8 which additionally comprises measuring the release of prostaglandin E2.

Fig. 1

Fig. 2

Fig. 3

HUMAN SKIN IRRITATION SCORES VERSUS
HUMAN SKIN CO-CULTURE CYTOTOXICITY (MTT-50)

Fig. 4

HUMAN SKIN IRRITATION SCORES VERSUS
HUMAN SKIN CO-CULTURE CYTOTOXICITY (LDH-50)

Fig. 5

EP 0 497 399 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CLINICAL RESEARCH vol. 38, no. 2, 1 February 1990, NEW YORK NY USA page 616; R. OSBORNE ET AL.: 'Evaluation of surfactant-induced toxicity in cultured skin cells.' *see whole abstract* | 1-9 | G01N33/50 |
| Y | DE-A-3 737 652 (BATELLE-INSTITUT) * the whole document * | 1-9 | |
| Y | US-A-4 016 036 (H. GREEN ET AL) * the whole document * | 1-9 | |
| T | TOXICOLOGY IN VITRO vol. 5, no. 5-6, 1 May 1991, LONDON UK pages 563 - 567; R. OSBORNE ET AL: 'In vitro skin irritation testing with human skin cell cultures.' * the whole document * | 1-9 | |

TECHNICAL FIELDS
SEARCHED (Int. Cl.5)

G01N
C12Q

The present search report has been drawn up for all claims

| Place of search | Date of completion of the search | Examiner |
|---|---|---|
| THE HAGUE | 20 MAY 1992 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)